# EUROPÄISCHE PATENTSCHRIFT

(11) **EP 1 135 689 B1**
(45) Veröffentlichungstag und Bekanntmachung des Hinweises auf die Patenterteilung: **25.02.2004**
(21) Anmeldenummer: 99958153.1
(22) Anmeldetag: 02.12.1999
(51) Int. Cl.: G01N 33/96, G01N 33/68, G01N 33/574

(54) **STABILISIERUNG VON CYTOKERATIN ENTHALTENDEN KALIBRATOREN**
STABILISATION OF CALIBRATORS CONTAINING CYTOKERATIN
STABILISATION D'ETALONS CONTENANT DE LA CYTOKERATINE

(30) Priorität: 03.12.1998 DE 19855819
(43) Veröffentlichungstag der Anmeldung: 26.09.2001
(73) Patentinhaber: Roche Diagnostics GmbH, 68298 Mannheim (DE)
(72) Erfinder: KYRIATSOULIS, Apostolos, D-82362 Weilheim (DE); PAPPERT, Gunter, D-82319 Starnberg (DE); MOESSNER, Ellen, D-74426 Buehlerzell (DE); FRANKEN, Norbert, D-82319 Starnberg (DE); ROTTMANN, Michael, D-82362 Weilheim (DE); BODENMUELLER, Heinz, D-82327 Tutzing (DE)
(86) Internationale Anmeldenummer: PCT/EP1999/009407
(87) Internationale Veröffentlichungsnummer: WO 2000/033085

(56) Entgegenhaltungen:
- WO-A-91/10139

## Beschreibung

Gegenstand der vorliegenden Erfindung sind Kalibratoren oder Eichlösungen, die auf einer Humanserum-Matrix basieren und die in einem Verfahren zum Nachweis von Cytokeratin eingesetzt werden, ein Verfahren zu deren Herstellung, ein Verfahren zur Stabilisierung von Cytokeratin in Humanserum sowie ein immunologisches Verfahren zum Nachweis von Cytokeratin in einer Probe.

Immunologische Nachweisverfahren haben in der Diagnostik eine große Bedeutung erlangt. Sie zeichnen sich durch eine hohe Spezifität und Sensitivität aus, so daß sie auch für den Nachweis von niedrig konzentrierten Analyten in biologischen Flüssigkeiten gut geeignet sind. Immunologische Nachweisverfahren sind insbesondere auf dem Gebiet der Infektionskrankheiten, der Fertilitäts- und Schilddrüsendiagnostik, bei Stoffwechselerkrankungen und der Diagnose von Tumorerkrankungen von großer Bedeutung. Zu den bisher bedeutendsten Tumormarkern zählen beispielsweise carcinoembryonales Antigen (CEA), Alpha-Fetoprotein (AFP), Prostata spezifisches Antigen (PSA) und Cytokeratine (CK).

Humane Cytokeratine sind Bausteine der Intermediär-Filamente, die im wesentlichen das Cytoskelett von Epithelzellen aufbauen. Es sind mehr als 19 Cytokeratine bekannt, von denen die Cytokeratine 1 bis 8 als basische und die Cytokeratine 9 bis 19 als saure Cytokeratine bezeichnet werden. Die Cytokeratine können sich in der Zelle zu Tetrameren zusammenlagern. Ein Tetramer besteht dabei aus jeweils zwei basischen und zwei sauren Cytokeratin-Molekülen. Durch lineare Aggregation von Tetrameren entstehen Filamente. Intakte Cytokeratin-Moleküle sind als integrale Bestandteile der Intermediär-Filamente epithelialer Zellen wasserunlöslich. Die Komplexität und Zusammensetzung der Cytokeratine ist unterschiedlich in den verschiedenen epithelialen Geweben, das heißt Epithelzellen haben für das jeweilige Gewebe typische Cytokeratin-Zusammensetzungen.

In der Tumordiagnostik sind insbesondere die löslichen Fragmente des Cytokeratin 19 (CK 19) relevant, die auch als CYFRA 21-1 bezeichnet werden. Eine im Vergleich zum gesunden Menschen erhöhte Konzentration an CYFRA 21-1 deutet auf das Vorliegen einer Tumorerkrankung hin. Erhöhte Werte wurden bisher bei den Tumoren Bronchialkarzinom, Ovarialkarzinom, Cervixkarzinom, Blasenkarzinom und bei Kopf-Hals-Tumoren gefunden. Die Hauptindikation von CYFRA 21-1 liegt in der Verlaufskontrolle nichtkleinzelliger Bronchialkarzinome.

Ein Verfahren zum Nachweis von CK 19 nach dem Prinzip des Sandwich-ELISAs wird beispielsweise in der EP-A-0 460 190 beschrieben. Hierzu wird die zu untersuchende Probe einer Körperflüssigkeit mit mindestens zwei Rezeptoren R 1 und R2 inkubiert, wobei R1 und R2 monoklonale Antikörper sind, die jeweils verschiedene Epitope des CK 19 erkennen. Einer der Antikörper ist mit Biotin markiert, der andere trägt eine andere Markierung. Der Sandwich-Komplex aus R1, CK 19 und R2 bindet an eine mit Streptavidin beschichtete Festphase. Nach Trennung der festen von der flüssigen Phase wird die Markierung in einer der beiden Phasen, bevorzugt in der Festphase gemessen. Anhand des erhaltenen Signals kann der Tumormarker CK 19 in der Probe nachgewiesen werden.

Bei der Durchführung eines solchen Tests ist es wichtig, den erhaltenen Meßwert zumindest qualitativ als positiv (Tumormarker ist vorhanden) oder negativ (Tumormarker ist nicht vorhanden) einzuordnen. Dies trifft insbesondere auf die Einordnung von Meßdaten, die mittels automatisierter Systeme erhalten werden, zu. Oftmals ist auch eine Quantifizierung der Konzentrationen an Tumormarker erwünscht oder erforderlich. Das Testsystem muß daher vor Durchführung der Messungen mit Reagenzien, die eine definierte Konzentration an Analyt enthalten, kalibriert werden. Diese definierten Reagenzien werden im folgenden als Kalibratoren bezeichnet. Die Begiffe Eichlösung, Eichstandard, Standardlösung oder Kontrolle werden synonym für den Begriff Kalibrator verwendet.

Eine wichtige Anforderung an einen Kalibrator ist eine hohe Stabilität. Zum einen muß die Richtigkeit des Tests, eine möglichst 100 %-ige Wiederfindung des Analyten im Kalibrator und ein gutes Signal-Rausch-Verhältnis sichergestellt sein. Zum anderen muß eine zuverlässige Reproduzierbarkeit des Bestimmungsergebnisses über einen längeren Zeitraum gewährleistet sein. Daher müssen Kalibratoren über einen Zeitraum von mehreren Wochen unempfindlich gegenüber ihren Umgebungsbedingungen, das heißt Temperatur, direkte Sonneneinstrahlung auf dem Labortisch, ph-Wert, Pufferbedingungen etc. sein. Bei ungünstigen Bedingungen besteht die Gefahr der Hydrolyse, Proteolyse oder Denaturierung des Kalibrators. Der Einsatz eines nicht mehr intakten Kalibrators würde zu Fehlmessungen führen.

Viele Tests werden in Serumproben durchgeführt. Um die Vergleichbarkeit der Messungen zu gewährleisten, sollte der Kalibrator daher ebenfalls auf Serummatrix aufgebaut sein. Je empfindlicher das verwendete Meßsystem ist, desto größer werden die Meßunterschiede aufgrund des Sprungs in der Matrix zwischen Probe und Kalibrator. Es hat sich gezeigt, daß Cytokeratin auf Serummatrix nicht stabil ist. Bei der Lagerung eines Cytokeratin enthaltenden Kalibrators in flüssigem Zustand wird das Cytokeratin zerstört und denaturiert, so daß der Einsatz eines solchen Kalibrators über einen längeren Zeitraum nicht möglich ist.

In der Vergangenheit wurde daher die Serummatrix durch eine künstliche Matrix ersetzt, die bezüglich ihrer Zusammensetzung dem Serum nachgeahmt ist. Die künstliche Matrix basiert im allgemeinen auf einem Puffer, der mit verschiedenen Salzen und Proteinen (zum Beispiel Rinderserumalbumin) versetzt ist, um das Milieu des natürlichen Humanserums bezüglich Salz- und Proteinkonzentration sowie pH-Wert möglichst gut nachzuahmen. Dadurch ist es zwar gelungen, den Analyten Cytokeratin im Kalibrator zu stabilisieren. Ein Nachteil dieser Vorgehensweise ist jedoch die nicht optimale Vergleichbarkeit, insbesondere bei empfindlichen Meßsystemen, da die Proben Humanserum als Basismatrix haben. Es gibt also einen Sprung in der Matrix. Bei Proben im niedrigen Meßbereich in der Nähe des Cut-off-Wertes kann es dadurch zu Fehlmessungen kommen. Dies könnte im Extremfall dazu führen, daß der für den Kalibrator auf Kunstmatrixbasis ermittelte Wert für ein positives Cytokeratin-Signal nicht mit einem für eine Humanserumprobe ermittelten Wert entspricht, selbst wenn der Analyt Cytokeratin jeweils in vergleichbarer Konzentration vorhanden ist.

Aufgabe war es daher, einen Kalibrator für Verfahren zum Nachweis von Cytokeratin bereitzustellen, der auf Serum- bevorzugt Humanserummatrix beruht und der über einen Zeitraum von mindestens mehreren Wochen auch bei ungünstigen Umgebungsbedingungen stabil ist.

Die Aufgabe wird gelöst durch einen Kalibrator, dessen wesentliche Bestandteile Serum, bevorzugt Humanserum, Cytokeratin und Aprotinin sind. Es hat sich überraschenderweise gezeigt, daß durch den Zusatz des Proteasehemmers Aprotinin das Cytokeratin wirksam stabilisiert werden kann. Somit ist es möglich, einen Kalibrator für Cytokeratin-Nachweisverfahren zur Verfügung zu stellen, der auf natürlicher Serummatrix-Basis und insbesondere auf Basis einer Humanserummatrix hergestellt ist. Damit wird ein Matrix-Sprung bei der Vermessung der tatsächlichen Serumproben vermieden.

Weiterhin hat es sich gezeigt, daß der erfindungsgemäße Kalibrator eine hohe Langzeitund Temperaturstabilität besitzt. So beträgt die Wiederfindungsrate für den im Kalibrator gelösten Analyten Cytokeratin, bevorzugt CK 19, in einem Immunoassay nach dem Sandwich-Prinzip nach dreiwöchiger Belastung des lyophilisierten Kalibrators bei 30 bis 40 °C und anschließendem Wiederauflösen nahezu 100 %. Das Cytokeratin wird also nicht zerstört und wird von den im Test eingesetzten Antikörpern auch nach der Zeit-Temperatur-Belastung noch immunologisch erkannt. Die zu Beginn des Stabilitätstests gemessenen Konzentrationen an Cytokeratin der verschiedenen Kalibratoren entsprechen im wesentlichen den Konzentrationen nach dem Belastungstest. Die Stabilität und Präzision des erfindungsgemäßen Kalibrators ist somit vergleichbar mit der Stabilität des entsprechenden Kalibrators auf Kunstmatrix-Basis. Auch bei Lagerung des Kalibrators bei 2 bis 8 °C in flüssiger oder rekonstituierter Form, was der üblichen Lagertemperatur von Reagenzien im Kühlschrank entspricht, konnte selbst nach 10 Wochen eine Wiederfindung des Cytokeratins von nahezu 100 % festgestellt werden. Das heißt, der erfindungsgemäße Kalibrator ist auch nach längerer Aufbewahrung im Kühlschrank noch ohne qualitative oder quantitative Einbußen zu verwenden.

Entscheidend für die wesentlich verbesserte Stabilität des erfindungsgemäßen Kalibrators ist der Protease-Inhibitor Aprotinin. Andere Proteasehemmer haben sich als nicht wirksam erwiesen. Bei Aprotinin handelt es sich um ein kommerziell erhältliches Polypeptid, das aus 58 Aminosäuren besteht. Es wirkt inhibierend auf die Gerinnungsfaktoren XIIa, XIa und VIIIa sowie Plasmin und Plasminaktivatoren, ferner Trypsin, Chymotrypsin und Kallikrein. Überraschenderweise haben sich andere bekannte Protease-Inhibitoren als nicht geeignet für die Stabilisierung des Kalibrators erwiesen. Versuche mit anderen Substanzen wie Detergenzien und Salzen haben nicht den gewünschten Stabilisierungseffekt erbracht.

Aprotinin wird vorzugsweise in einer Konzentration von mindestens 10 mg/l im Kalibrator eingesetzt. Die maximal mögliche Konzentration liegt dort, wo der Test gestört wird oder wo aufgrund der nicht mehr gegebenen Löslichkeit Trübung einsetzt. Besonders bevorzugt sind Konzentrationen zwischen 25 und 40 mg/l.

Der Kalibrator kann entweder in flüssiger Form oder als Lyophilisat bereitgestellt werden. Zur Herstellung des Lyophilisats werden zunächst alle flüssigen und festen Bestandteile miteinander vermischt bzw. gelöst und anschließend lyophilisiert. Das Lyophilisat wird dann letztendlich als Rekonstituat, das heißt wieder in Flüssigkeit aufgelöst, verwendet. Zur Rekonstitution, das heißt zum Wiederauflösen des Lyophilisats wird im allgemeinen destilliertes Wasser verwendet, da somit keine unerwünschten Ionen dem Kalibrator zugesetzt und insbesondere die Salzkonzentrationen nicht verfälscht werden. Die Menge an Wasser ist abhängig von der gewünschtem Cytokeratin-Konzentration bzw. vom gewünschten Abfüllvolumen.

Der erfindungsgemäße Kalibrator kann darüberhinaus auch übliche, dem Fachmann bekannte Substanzen wie Salze oder zusätzliche Konservierungsmittel enthalten. Beispielsweise werden bevorzugt N-Methylisothiazolon und Oxypyrion bevorzugt in den üblichen Konzentrationen von jeweils etwa 1 mg/l eingesetzt.

Bevorzugt kann der erfindungsgemäße Kalibrator in lyophilisierter Form bei 4 °C über mehrere Monate ohne Qualitätsverluste eingelagert werden. Das Einlagern kann über einen Zeitraum von bis zu 36 Monaten bei 4 °C erfolgen. In rekonstituierter Form kann der Kalibrator einige Monate bei 4 °C gelagert werden.

Als Serum wird bevorzugt cytokeratin-freies Humanserum eingesetzt. Dieses kann durch Prozessierung über Affinitätschromatographie erhalten werden, oder man setzt ein von vornherein cytokeratin-freies Serum, das durch entsprechende Screening-Verfahren ermittelt werden muss, ein.

Gegenstand der Erfindung ist außerdem ein Verfahren zur Herstellung des erfindungsgemäßen Kalibrators. Die Herstellung des Kalibrators erfolgt bevorzugt in folgenden Schritten
a) Mischen von Serum mit Aprotinin
b) Filtrieren der Lösung
c) Lösen des Cytokeratins in Wasser
d) Mischen der Lösung aus Schritt b) mit dem gelösten Cytokeratin aus Schritt c)
e) Lyophilisieren der Lösungen aus d)
f) vor Gebrauch Lyophilisat in Wasser auflösen.

Der pH-Wert kann falls erforderlich in Schritt d) auf pH 7 bis 8, bevorzugt 7,2 eingestellt werden.

Ein weiterer Gegenstand der Erfindung ist auch ein Verfahren zur Herstellung von stabilisiertem Cytokeratin, das dadurch gekennzeichnet ist, daß dem bevorzugt aufgereinigten Cytokeratin der Proteasehemmer Aprotinin zugesetzt wird. Bevorzugt wird das so hergestellte stabilisierte Cytokeratin in einem Kalibrator verwendet.

Ebenfalls ein Gegenstand der Erfindung ist die Verwendung von Aprotinin zur Stabilisierung von Cytokeratin, insbesondere von CK 19.

Ein Gegenstand der Erfindung ist auch ein Verfahren zur Stabilisierung von Cytokeratin in Serum, bevorzugt Humanserum, das dadurch gekennzeichnet ist, daß dem Serum Aprotinin, bevorzugt in einer Konzentration von mindestens 10 mg/l zugesetzt wird.

Ein weiterer Gegenstand der Erfindung ist ein immunologisches Verfahren zur Bestimmung von Cytokeratin in einer Probe, insbesondere zur Bestimmung von CK 19. Das Verfahren ist dadurch gekennzeichnet, daß das für die Probe bestimmte Signal mit dem Signal verglichen wird, das mit Hilfe des erfindungsgemäßen Kalibrators erhalten wird, wobei der Kalibrator mit dem gleichen Verfahren wie die Probe vermessen wird.

Bevorzugt wird ein solches Verfahren zur Bestimmung des Cytokeratins und bevorzugt von CK 19 und des Kalibrators in folgenden Schritten durchgeführt:
a) Umsetzen der Probe mit einem für Cytokeratin spezifischen ersten Bindepartner, der eine mit einer Festphase bindefahige Gruppe trägt, über die die Bindung an eine Festphase erfolgen kann,
b) Umsetzen dieser Lösung mit einem weiteren Bindepartner, der eine Markierung trägt
c) Bindung des gebildeten Immunkomplexes an eine Festphase, wobei die Festphase bereits in Schritt a) vorhanden sein kann
d) Trennung der festen von der flüssigen Phase
e) Detektion der Markierung in einer oder beiden Phasen.
f) Vergleich der Messwerte des Kalibrators mit dem Wert der Probe und Quantifizierung.

Das Verfahren kann auch als kompetitiver Test nach dem Fachmann bekannten Methoden durchgeführt werden.

Bei den Bindepartnern handelt es sich bevorzugt um mono- oder polyklonale Antikörper oder deren Fragmente wie F(ab')₂, Fab' oder Fab-Fragmente, die spezifisch Cytokeratin und insbesondere CK 19 immunologisch erkennen und binden. Die Herstellung der Antikörper erfolgt nach dem Fachmann geläufigen Verfahren. Umfaßt sind auch solche Antikörper, die durch Veränderung der Antikörper beispielsweise durch gentechnologische Maßnahmen hergestellt wurden. Der Begriff Antikörper schließt alle zuvor aufgeführten Bedeutungen für Bindepartner ein.

Der eine für Cytokeratin spezifische erste Bindepartner kann entweder direkt an die Festphase gebunden sein, oder die Bindung an die Festphase erfolgt indirekt über ein spezifisches Bindungssystem. Die direkte Bindung dieses Bindepartners an die Festphase erfolgt nach dem Fachmann bekannten Methoden. Wird die Bindung indirekt über ein spezifisches Bindungssystem durchgeführt, so ist der erste Bindepartner ein Konjugat, das aus einem Antikörper gegen Cytokeratin und einem Reaktionspartner eines spezifischen Bindungssystems besteht. Unter einem spezifischen Bindungssystem werden hier zwei Partner verstanden, die spezifisch miteinander reagieren können. Das Bindungsvermögen kann dabei auf einer immunologischen Reaktion oder auf einer anderen spezifischen Reaktion beruhen. Bevorzugt wird als spezifisches Bindungssystem eine Kombination von Biotin und Avidin oder Biotin und Streptavidin verwendet. Weitere bevorzugte Kombinationen sind Biotin und Antibiotin, Hapten und Anti-Hapten, Fc-Fragment eines Antikörpers und Antikörper gegen dieses Fc-Fragment oder Kohlenhydrat und Lectin. Einer der Reaktionspartner des spezifisch bindefähigen Paares ist dann Teil des Konjugates.

Der andere Reaktionspartner des spezifischen Bindungssystems für den ersten Bindepartner liegt als Beschichtung der festen Phase vor. Die Bindung des anderen Reaktionspartners des spezifischen Bindungssystems an ein unlösliches Trägermaterial kann nach den üblichen, dem Fachmann bekannten Methoden vorgenommen werden. Hierbei ist sowohl eine kovalente als auch eine adsorptive Bindung geeignet.

Als Festphase geeignet sind Reagenzgläschen oder Mikrotiterplatten aus Polystyrol oder ähnlichen Kunststoffen, die an der Innenoberfläche mit einem Reaktionspartner des spezifischen Bindungssystems beschichtet sind. Weiterhin geeignet und besonders bevorzugt sind teilchenförmige Substanzen, wie beispielsweise Latexpartikel, magnetische Partikel, Molekularsiebmaterialien, Glaskörperchen, Kunststoffschläuche und dergleichen. Auch poröse, schichtförmige Träger wie Papier können als Träger verwendet werden. Besonders bevorzugt werden magnetische Kügelchen, sogenannte Beads verwendet, die wiederum mit dem entsprechenden Bindepartner des oben beschriebenen spezifischen Bindesystems beschichtet sind. Diese Mikropartikel können dann nach Ablauf der Testreaktion für die Durchführung der Nachweisreaktion beispielsweise durch Filtration, Zentrifugation oder im Falle der magnetischen Partikel durch einen Magneten von der flüssigen Phase getrennt werden.

Die Detektion der spezifischen Bindereaktionen zwischen den Antikörpern gegen Cytokeratin und Cytokeratin kann auf verschiedene Weise erfolgen. Im allgemeinen ist ein Bindepartner der spezifischen Bindereaktion markiert. Übliche Markierungen sind Chromogene, Fluorophore, zur Chemi- oder Elektrochemilumineszenz fähige Substanzen, Radioisotope, Haptene, Enzymmarkierungen oder Substanzen, die wiederum ein spezifisches Bindungspaar bilden können wie beispielsweise Biotin/Streptavidin.

Als Proben für die Durchführung des Verfahrens zum Nachweis von Cytokeratin können alle dem Fachmann geläufigen biologischen Flüssigkeiten verwendet werden. Bevorzugt werden als Probe Körperflüssigkeiten wie Vollblut, Blutserum, Blutplasma, Urin oder Speichel, besonders bevorzugt Blutserum eingesetzt.

Die folgenden Beispiele erläutern die Erfindung weiter.

### Beispiel 1

### Herstellung des erfindungsgemäßen Kalibrators

Die angegebenen Identifikationsnummern (Id.-Nr.) bezeichnen Katalognummern aus dem Katalog der Boehringer Mannheim GmbH, Deutschland. Zur Herstellung von 1 Liter CK-Kalibrator (CK-Konzentration 40 ng/ml) werden miteinander gemischt:
A: Humanserum, käuflich erworben, affinitätschromatographisch cytokeratin-frei gemacht
B: Aprotinin 34 mg, Id.-Nr.: 0236632-001
C: N-Methylisothiazolon-HCL 1g, Id.-Nr.: 1085901-105
D: Oxypyrion 1g, Id.-Nr.: 1085913-05
E: Cytokeratin 7,31 g aus einer Stocklösung mit der Konzentration von 0.05 µg/ml mit 941 g A+B+C+D

Das verwendete humane Cytokeratin Cyfra 21-1 entspricht dem Cytokeratin aus der humanen Zellinie MCF-7 gemäß Cyfra 21-1 CalSet, Kalibrationsset für den Elecsys® CYFRA 21-1 Immunoassay der Boehringer Mannheim GmbH Deutschland, Id.-Nr. 1820974.

### Beispiel 2

### Belastung des Kalibrators bei 35 °C

Die Durchführung erfolgt nach dem Elecsys® Cyfra 21-1-Verfahren der Boehringer Mannheim GmbH (Id.-Nr. 1 820 966). Die Elecsys®-Testführung basiert auf der Biotin/Streptavidin-Technologie. Testprinzip ist ein 1-Schritt-Sandwich-ELISA mit zwei Antikörpern, die verschiedene Epitope auf dem Analyten (Cytokeratin) erkennen. Die Festphase wird von magnetischen, mit Streptavidin beschichteten Latexbeads gebildet, an die ein biotinylierter, für Cytokeratin spezifischer Antikörper bindet. Die Detektion des gebundenen Tumormarkers erfolgt nach der Trennung der festen von der flüssigen Phase durch die Messung der Elektrochemilumineszenz, die von einem zweiten für Cytokeratin spezifischen, mit einem Rutheniumkomplex markierten Antikörper ausgeht.

Folgende Einsatzstoffe werden verwendet:
R1 (erster Antikörper, biotinyliertes Fab-Fragment): MAK<CK19>M-KS19.1-Fab(DE)-Bi(DDS); Konzentration 1,5 µg/ml
R2 (zweiter Antikörper, ruthenyliertes IgG): MAK<CK19>M-BM19.21-IgG-BPRU); Konzentration 2,1 µg/ml

90 µl R1 und 90 µl R2 werden zusammen mit Streptavidin beschichteten Magnetbeads nach Herstellervorschriften des Gerätes Elecsys® 2010 der Boehringer Mannheim GmbH, Deutschland, zusammen inkubiert und vermessen.

Verglichen werden der Cytokeratin enthaltende erfindungsgemäße Kalibrator, der frisch gelöst wurde, und der erfindungsgemäße Kalibrator, der über drei Wochen in lyophilisiertem Zustand bei 35°C belastet und anschließend rekonstituiert wurde. Die Ergebnisse sind in Tabelle 1 dargestellt.

**Tabelle 1 (zu Beispiel 2):**

| Humanmatrix-Masterkalibratoren, frisch gelöst und nach 3-wöchiger Belastung bei 35 °C | | | | | |
|---|---|---|---|---|---|
| | **frisch gelöst** | | | **nach 3-wöchiger Belastung bei 35 °C** | |
| **MK** | **Konz. (ng/ml)** | **Wdf. (%)** | | **Konz. (ng/ml)** | **Wdf. (%) zu frisch** |
| | | | | | |
| **1** | 0,0 | / | | 0,0 | / |
| **2** | 0,88 | 100 | | 0,87 | **99** |
| **3** | 7,57 | 100 | | 7,10 | **94** |
| **4** | 18,13 | 100 | | 17,19 | **95** |
| **5** | 60,85 | 100 | | 57,62 | **95** |
| **6** | 125 | 100 | | 119 | **95** |
| **7** | 302 | 100 | | 296 | **98** |
| **8** | 450 | 100 | | 430 | **96** |
| **9** | 613 | 100 | | 604 | **98** |
| MK: Masterkalibrator: Kalibrator, mit dem eine neue Reagenziencharge vor Auslieferung geeicht wird Wdf: Wiederfindung bel: belastet | | | | | |

Es zeigt sich, daß auch der belastete Kalibrator eine gute, nahezu 100%-ige Wiederfindung zeigt. Die Abweichung beträgt jeweils unter 10 % (größte Abweichung: 6 %). Der erfindungsgemäße Kalibrator ist also gegenüber einer hohen Temperatur über einen längeren Zeitraum stabil und liefert auch nach der Belastung zuverlässige Eichwerte. Auch bei versehentlicher falscher Lagerung des Kalibrators kann dieser bedenkenlos ohne Qualitätseinbußen weiterverwendet werden.

### Beispiel 3

### Langzeitbelastung des Kalibrators bei 4°C (flüssig)

Verglichen werden frisch gelöster Kalibrator und Kalibrator, der vor der Messung in flüssiger Form als Rekonstituat bei 4°C über 10 Wochen aufbewahrt wurde. Die Durchführung der Messungen erfolgt wie unter Beispiel 2 beschrieben. Die Ergebnisse sind in Tabelle 2 dargestellt.

**Tabelle 2 (zu Beispiel 3):**

| Humanmatrix-Standards, frisch gelöst und nach 10-wöchiger Lagerung bei 4 °C | | | | | |
|---|---|---|---|---|---|
| | **frisch gelöst** | | | **nach 10-wöchiger Lagerung bei 4°C** | |
| **Standard** | **Konz. (ng/ml)** | **Wdf. (%)** | | **Konz. (ng/ml)** | **Wdf. (%) zu frisch** |
| | | | | | |
| **A** | 0,20 | / | | 0,44 | / |
| **B** | 3,33 | 100 | | 3,13 | **94** |
| **C** | 8,00 | 100 | | 7,85 | **98** |
| **D** | 18,84 | 100 | | 17,81 | **95** |
| **E** | 54,52 | 100 | | 51,68 | **95** |
| **F** | 100,89 | 100 | | 95,53 | **95** |

Auch in flüssiger Form zeigen sich beim erfindungsgemäßen Kalibrator nach einer Belastung über 10 Wochen bei 4°C gute Ergebnisse bezüglich Signal- und Konzentrationswiederfindung.

### Beispiel 4

### Langzeitstabilität des Kalibrators: 24 Monate bei 4°C / 24 Monate - 20°C

Verglichen werden der Kalibrator, der vor der Messung über 24 Monate bei 4°C gelagert wurde, mit Kalibrator, der über 24 Monate bei -20°C gelagert wurde (jeweils als Lyophilisat). Die Durchführung erfolgt wie in Beispiel 2 beschrieben. Die Ergebnisse sind in Tabelle 3 dargestellt.

**Tabelle 3 (zu Beispiel 4)**

| Humanmatrix-Kalibratoren nach 24-monatiger Lagerung bei -20 °C und +4 °C | | | | | |
|---|---|---|---|---|---|
| | **24 Monate bei -20 °C** | | | **24 Monate bei +4 °C** | |
| **Standard** | **Konz. (ng/ml)** | **Wdf. (%)** | | **Konz. (ng/ml)** | **Wdf. (%) zu frisch** |
| | | | | | |
| **MK 1** | 0,0 | / | | 0,0 | / |
| **MK 2** | 3,71 | 100 | | 3,85 | **104** |
| **MK 3** | 9,01 | 100 | | 9,04 | **100** |
| **MK 4** | 40,11 | 100 | | 39,82 | **99** |
| **MK 5** | 394,8 | 100 | | 395.0 | **100** |

Für beide Belastungsmethoden zeigt sich eine gute Wiederfindung. Der cytokeratinhaltige Kalibrator kann in lyophilisierter Form ohne Bedenken über Zeiträume einiger Jahre bei + 4 bzw. - 20 °C gelagert werden, ohne daß die Qualität signifikant leidet.

### Beispiel 5

### Vergleich der Messwerte von Tumormarkerkontrollen:

### Eichung des Messgerätes auf Kunstmatrix- und Humanserum-Basis

Vor Durchführung der Messungen wird das Elecsys®-Messgerät mit dem Cytokeratin enthaltenden Kalibrator aufKunstmatrix-Basis geeicht und anschließend die Tumormarkerkontrollen anhand dieser Eichwerte vermessen. Die für diese Messung gefundenen Werte werden definitionsgemäß auf 100% Wiederfindung gesetzt. Anschließend wird das gleiche Gerät mit dem erfindungsgemäßen Kalibrator auf Humanserum-Basis geeicht und wiederum die Tumormarkerkontrollen anhand dieser Eichwerte vermessen. Die Ergebnisse sind in Tabelle 4 dargestellt.

Die Tumormarkerkontrollen TMK I bzw. II und PCT I bzw. II basieren auf prozessiertem Humanserum und enthalten neben anderen Tumormarkern wie beispielsweise PSA und AFP das Cytokeratin Cyfra 21-1 mit Sollwerten für zwei Konzentrationsbereiche (I und II).

**Tabelle 4 (zu Beispiel 5)**

| Vergleich zwischen Tumormarkerkontrollen; Eichung für Cytokeratin auf Kunstmatrix-Basis im Vergleich zur Eichung auf Humanserum-Basis | | | | | |
|---|---|---|---|---|---|
| | **Eichung mit Kalibrator auf Kunstmatrix-Basis** | | | **Eichung mit Kalibrator auf Humanserum-Basis** | |
| **Tumor-marker-kontrolle** | **Konz. (ng/ml)** | **Wdf. (%)** | | **Konz. (ng/ml)** | **Wdf. (%)** |
| | | | | | |
| **TMKI** | 4,63 | 100 | | 5,07 | **110** |
| **TMK II** | 28,5 | 100 | | 29,8 | **105** |
| **PCTI** | 4,99 | 100 | | 5,23 | **105** |
| **PCT II** | 30,7 | 100 | | 31,7 | **103** |

Es kann gezeigt werden, daß die Abweichungen für die Messungen der Tumormarkerkontrollen basierend auf den Eichungen mit den Kalibratoren auf Kunst- bzw. Humanmatrix-Basis nur maximal 10 % betragen. Die für die Tumormarkerkontrollen erhaltenen Werte sind somit unabhängig von dem für die Eichung des Gerätes verwendeten Kalibrator. Der Kalibrator auf Humanserum-Basis kann also den Kalibrator auf Kunstmatrix-Basis ersetzen.

## Patentansprüche

1. Kalibrator für ein Verfahren zum Nachweis von Cytokeratin in einer Probe enthaltend Serum, Cytokeratin und Aprotinin.

2. Kalibrator nach Anspruch 1, **dadurch gekennzeichnet, daß** als Serum Humanserum verwendet wird.

3. Kalibrator nach Anspruch 1, **dadurch gekennzeichnet, daß** das zugesetzte Cytokeratin CK 19 ist.

4. Kalibrator nach Anspruch 1, **dadurch gekennzeichnet, daß** darin Aprotinin in einer Konzentration von mindestens 10 mg pro Liter enthalten ist.

5. Verfahren zur Herstellung eines Kalibrators gemäß Anspruch 1, umfassend die Schritte
a) Mischen von Serum mit Aprotinin
b) Filtrieren der Lösung
c) Lösen des Cytokeratins in Wasser
d) Mischen der Lösung aus Schritt b) mit dem gelösten Cytokeratin aus Schritt c)
e) Lyophilisieren der Lösungen aus d)
f) vor Gebrauch Lyophilisat in Wasser auflösen.

6. Verfahren zur Stabilisierung von Cytokeratin in Serum, **dadurch gekennzeichnet, daß** dem Serum Aprotinin zugesetzt wird.

7. Verfahren zur Herstellung von stabilisiertem Cytokeratin, **dadurch gekennzeichnet, daß** dem aufgereinigten Cytokeratin Aprotinin zugesetzt wird.

8. Verwendung von Aprotinin zur Stabilisierung von Cytokeratin.

9. Immunologisches Verfahren zur Bestimmung von Cytokeratin in einer Probe, **dadurch gekennzeichnet, daß** das für die Probe bestimmte Signal mit dem Signal verglichen wird, das mit Hilfe des Kalibrators gemäß den Ansprüchen 1 bis 4 erhalten wird, wobei der Kalibrator mit dem gleichen Verfahren wie die Probe vermessen wird.

## Claims

1. Calibrator for a method to detect cytokeratin in a sample containing serum, cytokeratin and aprotinin.

2. Calibrator as claimed in claim 1, wherein human serum is used as the serum.

3. Calibrator as claimed in claim 1, wherein the added cytokeratin is CK 19.

4. Calibrator as claimed in claim 1, wherein it contains aprotinin at a concentration of at least 10 mg per litre.

5. Process for producing a calibrator as claimed in claim 1 comprising the steps:
a) mixing serum with aprotinin
b) filtering the solution
c) dissolving the cytokeratin in water
d) mixing the solution from step b) with the dissolved cytokeratin from step c)
e) lyophilizing the solutions from d)
f) dissolving the lyophilisate in water before use.

6. Method for stabilizing cytokeratin in serum, wherein aprotinin is added to the serum.

7. Process for producing stabilized cytokeratin, wherein aprotinin is added to purified cytokeratin.

8. Use of aprotinin to stabilize cytokeratin.

9. Immunological method for determining cytokeratin in a sample, wherein the signal determined for the sample is compared with the signal that is obtained with the aid of the calibrator as claimed in claims 1 to 4, the calibrator being measured with the same method as for the sample.

## Revendications

1. Calibrateur pour un procédé de détection de cytokératine dans un échantillon contenant du sérum, de la cytokératine et de l'aprotinine.

2. Calibrateur selon la revendication 1, **caractérisé en ce que** l'on utilise comme sérum du sérum humain.

3. Calibrateur selon la revendication 1, **caractérisé en ce que** la cytokératine ajoutée est CK 19.

4. Calibrateur selon la revendication 1, **caractérisé en ce que** l'aprotinine y est contenue en une concentration d'au moins 10 mg/l.

5. Procédé de préparation d'un calibrateur selon la revendication 1, comprenant les étapes consistant à :
a) mélanger le sérum avec l'aprotinine,
b) filtrer la solution,
c) dissoudre la cytokératine dans de l'eau,
d) mélanger la solution provenant de l'étape b) avec la cytokératine dissoute provenant de l'étape c),
e) lyophiliser les solutions provenant de d),
f) dissoudre le lyophilisat dans de l'eau avant emploi.

6. Procédé de stabilisation de cytokératine dans du sérum, **caractérisé en ce que** de l'aprotinine est ajoutée au sérum.

7. Procédé de préparation de cytokératine stabilisée, **caractérisé en ce que** de l'aprotinine est ajoutée à la cytokératine épurée.

8. Utilisation de l'aprotinine pour stabiliser de la cytokératine.

9. Procédé immunologique pour déterminer une cytokératine dans un échantillon, **caractérisé en ce que** le signal déterminé pour l'échantillon est comparé avec le signal qui est obtenu à l'aide du calibrateur selon les revendications 1 à 4, le calibrateur étant mesuré avec le même procédé que celui pour l'échantillon.
